# EUROPEAN PATENT APPLICATION

(11) **EP 4 356 909 A1**
(43) Date of publication of application: **24.04.2024**
(21) Application number: 22382994.6
(22) Date of filing: 17.10.2022
(51) Int. Cl.: A61K 31/40, A61K 31/4192, C07D 403/12, A61P 35/00, A61P 37/02, A61P 25/00, A61P 21/00, A61P 9/00

(54) **1-(SULFONYL)-N-PHENYLPYRROLIDINE-2-CARBOXAMIDES DERIVATIVES AND USE THEREOF**

(71) Applicant: Selabtec Sciences, SLU, 08225 Terrassa (ES)
(72) Inventor: PÉREZ OZCÁRIZ, Sergio, TERRASSA (ES); CASTELLS BOLIART, Josep, TERRASSA (ES); PASCUAL GILABERT, Marta, TERRASSA (ES)
(74) Representative: Ponti & Partners, S.L.P

(57) **Abstract**

The present invention relates to 1-(sulfonyl)-N-phenylpyrrolidine-2-carboxamides derivatives according to formula (I) for use as a calcineurin inhibitor. The present invention further relates to new compounds included in such formula (I).

## Description

### Field of the invention

The present invention relates to medicinal chemistry. In particular the present invention relates to 1-(sulfonyl)-N-phenylpyrrolidine-2-carboxamides derivatives represented by formula (I) for use as a calcineurin inhibitor. The present invention further relates to new compounds included in such formula (I).

### Background

The immune system is a complex network of cells and molecules that work together to protect the body against various diseases. Hyperactivity or inappropriate activity of the immune system is a serious and widespread medical problem. It contributes to acute and chronic immune diseases, e.g., allergic and atopic diseases, asthma, allergic rhinitis, allergic conjunctivitis and atopic dermatitis, and in certain circumstances, the immune system may also attack and damage the body's own tissues, organs and cells, resulting in autoimmune diseases (ADs).

Almost any part of the body can be targeted by the immune system, including the heart, brain, nerves, muscles, skin, eyes, lungs, the digestive tract and blood vessels. A broad range of ADs exist given that they vary according to the part of the body that is being targeted by the immune system. Currently, more than 100 different types of ADs have been identified. Common ADs include rheumatoid arthritis, systemic lupus erythematosus, multiple sclerosis, type 1 diabetes, psoriasis, and celiac diseases (for a complete list of said ADs, see WO 2017/212018 (pages 3-7)). In fact, ADs affect 5 to 10% of the global population, particularly women, who are two to ten times more likely to suffer from an AD than men. Some of the ADs are within the top 10 leading causes of death among women aged 65 and older (Autoimmune Diseases - Modern Diseases, Study for the ENVI Committee, European Parliament, 2017, and references cited therein, available at http://www.europarl.europa.eu/cmsdata/133620/ENVI%202017-09%20WS%20Autoimmune%20diseases%20%20PE%20614.174%20(Publication).pdf).

Hyperactivity or inappropriate activity of the immune system is also involved in transplant graft rejections and graft-versus-host disease. Rejection takes place when the organ recipient's immune system recognizes the donor organ as foreign and attempts to eliminate it. Common organs transplanted include, but are not limited to, kidney, liver, heart, lung, and pancreas, among others. Accordingly, immunosupressive drugs prevent the graft vs host reactions or tissue rejections after allo- or xenotransplantation.

A certain family of transcription factors, the NFAT proteins (also named NFATc), are expressed in immune cells and play a key role in eliciting immune responses. The NFAT proteins are activated by calcineurin, and the activated NFAT proteins, in turn, induce transcription of cytokine genes which are required for an immune response. The immunosuppressive drugs cyclosporin A, tacrolimus (FK506) and voclosporin are potent inhibitors of cytokine gene transcription in activated immune cells, and have been reported to act by inhibiting calcineurin such that calcineurin is not able to activate NFAT. These drugs, however, can display nephrotoxic and neurotoxic effects after long term usage. Since calcineurin is ubiquitously expressed in many tissues, the drugs' inhibition of calcineurin activity toward substrates other than NFAT may contribute to the observed toxicity.

Calcineurin (CN or PPP3, formerly PP2B) is a serine/threonine protein phosphatase regulated by Ca²+ and calmodulin. CN regulates development, memory, cardiac function, and immune response by dephosphorylating a variety of protein substrates, including the family of cytosolic Nuclear Factor of Activated T-cells (NFATc) transcription factors. NFATc proteins have been shown to be direct substrates of calcineurin. Dephosphorylated NFATc proteins translocate into the nucleus where, in cooperation with other transcription factors, induce the expression of many cytokine and chemokine genes, leading to T cell activation.

Calcineurin plays a prominent role in many physiological processes, including homeostasis, angiogenesis, myogenesis, adipogenesis, osteogenesis, chondrocyte differentiation, cardiovascular system development, pancreatic β-cell proliferation, hair follicle cell differentiation and remodelling, and activities of cells of the immune and nervous systems, including schizophrenia, diabetes mellitus, Down synthdrome, cardiac hypertrophy, as well as in neurodegenerative diseases, such as Alzheimer's disease, psychotic disorders, epilepsy, among others (Erdmann, F et al. Calcineurin inhibitors: status quo and perspectives. BioMol Concepts, 2011, vol. 2, pp. 65-78; Kipanyula, M.J. et al The emerging roles of the Calcineurin-Nuclear Factor of activated T-Lymphocyte pathway in nervous system functions and diseases, J. Aging Research, 2016, Article ID 5081021, http://dx.doi.org/10.1155/2016/5081021).

The immunosuppressant drugs cyclosporin A (CsA) and tacrolimus (FK506) are the cornerstone of immunosuppressive therapy. These drugs, in complex with endogenous immunophilins, sterically hinder the phosphatase activity of CN towards NFATc and all other substrates, by blocking the peptide LxVP binding site of CN. Both drugs have narrow therapeutic windows. As a consequence, despite their benefits, severe side effects, such as neurotoxicity, renal dysfunction, hypertension and diabetes, have been related to long-term administration (Review: Azzi, J. R. et al. Calcineurin Inhibitors: 40 Years Later, Can't Live Without... J. Immunol. December 15, 2013, 191 (12) 5785-5791; DOI: https://doi.org/10.4049/jimmunol.1390055).

Siebert, M et al (Novel inhibitors of the calcineurin/NFATc hub-aternatives to CsA and FK506? Cell Communication and Signaling, 2009, 7:25 doi:10.1189/1478-811X-7-25) and Erdmann, F *et al* (vide supra) review novel inhibitors of the calcineurin/NFATc signalling pathway. Moreover, they disclose that compounds that are able to discriminate not only between the inhibition of calcineurin and PPlases (peptidiyl-prolyl *cis-trans* isomerase) as well as of NFATc and other substrates of calcineurin might cause fewer side effects in clinical applications. Not a single one of the substances disclosed therein have been introduced in clinical trials so far.

According to the prior art, the CN docking site of NFATc is mainly formed by the PxlxlT motif, the main anchoring site, and the LxVP motif, to properly position the substrate phosphoresidue towards the catalytic active site (Li, H. et al. Interaction of calcineurin with substrates and targeting proteins. Trends in Cell Biology, 2011, 21 (2), 91-103; Matsoukas, Minos et al (2015). Identification of small-molecule inhibitors of calcineurin-NFATc signaling that mimic the PxlxlT motif of calcineurin binding partners. Science Signaling. 8. 10.1126/scisignal.2005918 & Fig. S1 in supplementary information).

Perez-Riba et al (WO2016207212 A1) and Matsoukas *et al* (vide supra) disclose non-peptide inhibitors of the calcineurin -NFAT signalling pathway that disrupt the CNA-NFTAc interaction without blocking general calcineurin phosphatase activity through specifically binding of the compounds disclosed therein to the PxlxlT binding region of calcineurin.

WO 02/102380 A1 discloses monocyclic or bicyclic carbocycles and heterocycles which are useful as inhibitors of factor Xa for the treatment and prevention of thromboembolic disorders.

US 2004/0006062 discloses sylfonylaminovalerolactams and derivatives thereof useful as inhibitors of trypsin-like serine proteases, specifically factor Xa. These compounds do not include the core structure contained in the compounds of the formula (I) of the present invention.

EP3587416A1 discloses 2-oxopiperidin-3-yl derivatives and use thereof as calcineurin inhibitors. These compounds do not include the core structure contained in the compounds of the formula (I) of the present invention.

WO 02/00651 discloses inhibitors of trypsin-like serine protease enzymes, especially factor Xa as anticoagulant agents for treatment and prevention of thromboembolic disorders.

US8455660B2 discloses compounds 1-(sulfonyl)-*N*-phenylpyrrolidine-2-carboxamides with the potential use for drug discovery applications and the synthetic methods are also exposed.

Therefore, it is necessary to develop alternative strategies for disrupting Ca2+-calcineurin-NFATc signalling pathway that overcome the problems associated to the methods based on the use of currently available immunosupressants to be more specific for the calcineurin/NFAT pathway. Additionally, there is a need for immunosuppressive agents which selectively inhibit the calcineurin-NFAT interactions and which do not inhibit the enzymatic activity of calcineurin for its other substrates.

Applicants of the present invention have surprisingly found that compounds of formula (I) compete with NFATc binding to CN by a different site from the previously described PxIxIT-and LxVP- binding motifs on CNA. The above mentioned pocket may be considered as a novel binding site for calcineurin inhibition.

### Summary of the invention

In a first aspect, the present invention relates to 1-(sulfonyl)-N-phenylpyrrolidine-2-carboxamides derivatives represented by formula (I) for use as a calcineurin inhibitor in the prevention and/or treatment of a disease or disorder related thereto, preferably, for the prevention and/or treatment of a disease or disorder selected from an autoimmune disease, organ transplant rejection, cancer, heart-related disease or disorder, muscle-related disease or disorder, and neurological disease or disorder.

In a second aspect, the present invention relates to new compounds included in the formula (I).

### Brief description of the drawings

Figure 1. Effect of INK compounds (see reference in the examples) on NFATc activation in HEK 293T cells.
Figure 2: INK compounds (see reference in the examples) do not affect CN phosphatase activity towards the pNPP (p-nitrophenyl phosphate) substrate.
Figure 3: Compounds do not inhibit calcineurin phosphatase activity towards the RII substrate
Figure 4: Synthesis scheme of the compound SLB-605.
Figure 5: Assay of compound SLB-605 against rhabdomyosarcoma cell lines (RD (a) and RH4 (b))
Figure 6: Assay of compound SLB-605 against neuroblastoma cell lines (SKNBE2 (a) and SHSY5Y (b)).
Figure 7: Assay of compound SLB-605 against osteoblastoma cell line.

### Detailed description

The present invention relates in a first aspect to a 1-(sulfonyl)-N-phenylpyrrolidine-2-carboxamides derivative or herein also named with the term "compound" represented by formula I: 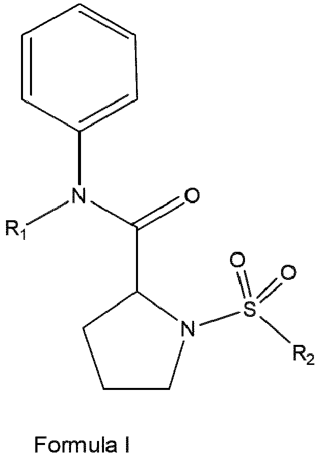 and stereoisomers and pharmaceutically acceptable salts thereof, wherein
R¹ is hydrogen; alkyl optionally substituted with Het; benzoyl optionally substituted with alkyl or cyano; or Het;
R² is aryl optionally substituted with 1, 2, or 3 substituents each independently selected from alkyl, halo, cyano, amide, and carboxyl; or benzyl optionally substituted with 1, 2, or 3 substituents each independently selected from alkyl, halo, cyano, amide, and carboxyl; wherein each Het as a group or part of a group is a monocyclic ring with 5 or 6 atoms; or a bicyclic ring structure consisting of a 6 membered ring fused to a 4, 5, or 6 membered ring; each of the rings (monocyclic or bicyclic) being saturated, partially unsaturated, or completely unsaturated, wherein at least one of the rings contains 1 to 4 heteroatoms each independently selected from nitrogen, oxygen and sulphur; and any of the rings (monocyclic or bicyclic) is optionally substituted with one, two or three substituents each independently selected from the group consisting of halo, hydroxyl, nitro, cyano, C₁₋₆alkyl, and C₁₋₆alkoxy. for use as a calcineurin inhibitor in the prevention and/or treatment of a disease or disorder related thereto.

In a preferred embodiment, in the compound of formula (I) for use as a calcineurin inhibitor as shown above:
R¹ is hydrogen; C₁₋₆alkyl optionally substituted with Het; benzoyl optionally substituted with C₁₋₆alkyl or cyano; or Het.
R² is aryl optionally substituted with 1, 2, or 3 substituents each independently selected from C₁₋₆alkyl, halo, cyano, amide, and carboxyl; or benzyl optionally substituted with 1, 2, or 3 substituents each independently selected from C₁₋₆alkyl, halo, cyano, and carboxyl. wherein each Het as a group or part of a group is a monocyclic ring with 5 or 6 atoms; or a bicyclic ring structure consisting of a 6 membered ring fused to a 5 or 6 membered ring; each of the rings being saturated, partially unsaturated, or completely unsaturated, wherein at least one of the rings contains 1 to 4 heteroatoms each independently selected from nitrogen, oxygen and sulphur; and any of the rings is optionally substituted with one, two or three substituents each independently selected from the group consisting of halo, hydroxyl, cyano, and C₁₋₆alkyl.

In a further preferred embodiment, in the compound of formula (I) for use as a calcineurin inhibitor as shown above:
R¹ is hydrogen; methyl optionally substituted with Het; or benzoyl optionally substituted with cyano;
R² is aryl optionally substituted with 1, 2, or 3 substituents each independently selected from methyl, halo, cyano, amide, and carboxyl; or benzyl optionally substituted with 1, 2, or 3 substituents each independently selected from methyl, halo, and cyano.
each Het as a group or part of a group is a bicyclic ring structure consisting of a 6 membered ring fused to a 5 or 6 membered ring; each of the rings being partially unsaturated; wherein at least one of the rings contains 1 to 4 heteroatoms each independently selected from nitrogen, oxygen and sulphur.

In a yet further preferred embodiment, in the compound of formula (I) for use as a calcineurin inhibitor as shown above:
R¹ is selected from: 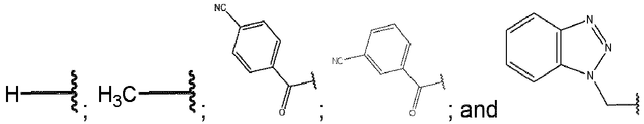
R² is selected from:

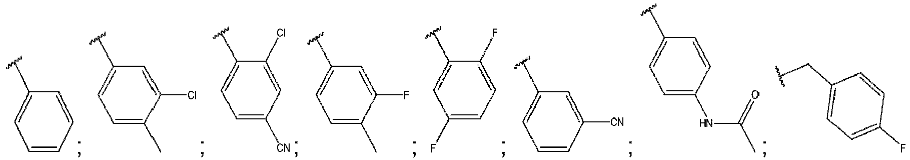

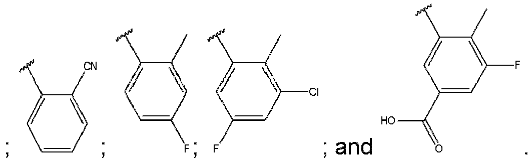

As used in the foregoing and hereinafter, the following definitions apply unless otherwise noted.

The term "halo" is generic to fluoro, chloro, bromo and iodo.

The terms "heterocyclic ring" or "heterocycle" are used interchangeably herein and refer to any compound in which plurality of atoms form a ring via a plurality of covalent bonds, wherein the ring includes at least one atom other than a carbon atom. Particularly contemplated heterocyclic bases include 4-, 5- and 6-membered rings containing at least 1 to 4 heteroatoms each independently selected from nitrogen, oxygen and sulphur as the non-carbon atom (e.g., imidazole, pyrrole, triazole, dihydropyrimidine). Further contemplated heterocycles may be fused (i.e., covalently bound) to another ring or heterocycle, and are thus termed "fused heterocycle" or "fused heterocyclic base" as used herein. Especially contemplated fused heterocycles include a 5-membered ring fused to a 6-membered ring (e.g., purine, pyrrolo[2,3-d]pyrimidine), and a 6-membered ring fused to another 6-membered or higher ring (e.g., pyrido[4,5-d]pyrimidine, benzodiazepine). Still further contemplated heterocyclic bases may be aromatic, or may include one or more double or triple bonds. Moreover, contemplated heterocyclic bases and fused heterocycles may further be substituted in one or more positions. And any one of the rings being optionally substituted with one, two or three substituents each independently selected from the group consisting of halogen, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, and C₃₋₇cycloalkyl.

"Het" is defined herein as a group or part of a group being a monocyclic ring with 5, 6 or 7 ring atoms optionally fused to an aryl or a bicyclic ring structure comprising a 5, 6 or 7 membered ring linked to a 4, 5, or 6 membered ring; each of the rings being saturated, partially unsaturated, or completely unsaturated; at least one of the rings containing 1 to 5 heteroatoms each independently selected from nitrogen, oxygen and sulphur; and any one of the rings being optionally substituted with one, two, three or four substituents each independently selected from the group consisting of OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NO₂; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

For therapeutic use, salts the compounds of formula (I) are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the scope of the present invention.

The pharmaceutically acceptable acid and base addition salts as mentioned above are meant to comprise the therapeutically active non-toxic acid and base addition salt forms which the compounds of formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The compounds of formula (I) containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which either the compounds of formula (I) as well as the salts thereof, are able to form. Such solvates are for example hydrates, alcoholates and the like.

The term "quaternary amine" as used hereinbefore defines the quaternary ammonium salts which the compounds of formula (I) are able to form by reaction between a basic nitrogen of either the compounds of formula (I) or the compounds of formula (I)I and an appropriate quatemizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The N-oxide forms of the present compounds are meant to comprise the compounds of formula (I) wherein one or several nitrogen atoms are oxidized to the so-called N-oxide.

It will be appreciated that the compounds of formula (I) may have metal binding, chelating, complex forming properties and therefore may exist as metal complexes or metal chelates.

Such metalated derivatives of the compounds of formula (I) are intended to be included within the scope of the present invention.

Some of the compounds of the compounds of formula (I) may also exist in their tautomeric form. Such forms although not explicitly indicated in the above formula are intended to be included within the scope of the present invention.

The compounds described herein may have asymmetric centers and occur as racemates, racemic mixtures, individual diastereomers or enantiomers, with all isomeric forms being included in the present invention. Compounds of the present invention having a chiral center can exist in and be isolated in optically active and racemic forms. Some compounds can exhibit polymorphism.

The term "alkyl" as used herein it does refer to any linear, branched, or cyclic hydrocarbon in which all carbon-carbon bonds are single bonds. Alkyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR₆R₇; CN; COR₆; CONR₆R₇; CO₂R₆; C(S)OR₆; OCONR₆R₇; OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "alkenyl" and "optionally substituted alkenyl" are used interchangeably herein and refer to any linear, branched, or cyclic alkyl with at least one carbon-carbon double bond. Alkenyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR₆R₇; CN; COR₆; CONR₆R₇; CO₂R₆; C(S)OR₆; OCONR₆R₇;OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

Furthermore, the term "alkynyl" and "optionally substituted alkynyl" are used interchangeably herein and refer to any linear, branched, or cyclic alkyl or alkenyl with at least one carbon-carbon triple bond. Alkynyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR₆R₇; CN; COR₆; CONR₆R₇; CO₂R₆; C(S)OR₆; OCONR₆R₇;OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "aryl" as used herein is a group or part of a group being phenyl, naphthalenyl, anthracenyl, phenantrenyl, biphenyl, optionally fused to Het; each optionally substituted with one, two, three or four substituents selected from OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "substituted" as used herein refers to a replacement of an atom or chemical group (e.g., H, NH₂, or OH) with a functional group, and particularly contemplated functional groups include nucleophilic groups (e.g., -NH₂, -OH, -SH, -NC, etc.), electrophilic groups (e.g., C(O)OR, C(X)OH, etc.), polar groups (e.g., -OH), non-polar groups (e.g., aryl, alkyl, alkenyl, alkynyl, etc.), ionic groups (e.g., -NH₃⁺), and halogens (e.g., -F, -CI), and all chemically reasonable combinations thereof. Thus, the term "functional group" and the term "substituent" are used interchangeably herein and refer to nucleophilic groups (e.g., -NH₂, - OH, -SH, -NC, -CN, etc.), electrophilic groups (e.g., C(O)OR, C(X)OH, C(Halogen)OR, etc.), polar groups (e.g., -OH), non-polar groups (e.g., aryl, alkyl, alkenyl, alkynyl, etc.), ionic groups (e.g., -NH₃⁺), and halogens.

Functional groups such as -OH, -NH₂, and the like, can incorporate protecting groups (abbreviated as PG) such as those known for those skilled in the art (GREENE'S PROTECTIVE. GROUPS IN ORGANIC. SYNTHESIS. Fourth Edition. PETER G. M. WUTS. and. THEODORA W. GREENE. 2007. Wiley-Interscience). By way of example, hydroxyl protection (Greene's, *vide supra,* pages 16-366), including 1,2-diols could be in the form of ethers, esters, cyclic acetals, cyclic ketals, and silyl derivatives, such as, but not limited to, methyl ether, methoxymethyl ether, methylthiomethyl ether, *t*-butylthiomethyl ether, (phenyldimethylsislymethoxymethyl) ether, benzyloxymethyl ether, p-methoxybenzyloxymethyl ether, p-nitrobenzyloxymethyl ether, o-nitrobenzyzloxymethyl ether, (4-methoxyphenoxy)methyl ether, guaiacolmethyl ether, *t*-butoxymethyl ether, 4-pentenyloxymethyl ether, siloxymethyl ether, 2-methoxethoxymethyl ether, 2,2,2-trichloroethoxymethyl ether, bis(2-chloroethoxy)methyl ether, 2-(trimethylsilyl)ethoxymethyl ether, menthoxymethyl ether, tetrahydropyranyl ether, 3-bromotetrahydropyranyl ether, tetrahydrothiopyranyl ether, 1-methoxycyclohexyl ether, 4-methoxytetrahydropyranyl ether, 4-methoxytetrahydrothiopyranyl ether, 4-methoxytetrahydrothiopyranyl S, S-Dioxido ether, 1-[(2-chloro-4-methyl)phenyl]-4-methoxypiperidin-4-yl ether, 1,4-dioxan-2-yl ether, 1,4-dioxan-2-yl ether, tetrahydrothiofuranyl ether, 2,3,3a,4,5,6,7,7a-octahydro-7,8,8-trimethyl-4,7-methanobenzofuran-2-yl ether, 1-ethoxyethyl ether, 1-(2-chloroethoxy)ethyl ether, 1-hydroxyethyl ether, 2-bromoethyl ether, 1-[2-(trimethylsilyl)ethoxy]ethyl ether, 1-(2-cyanoethoxy)ethyl ether, prenyl ether, cinnamyl ether, propargyl ether, p-nitrophenyl ether, 1-methyl-1-methoxyethylether, 1-methyl-1-benzyloxyethyl ether, 1-methyl-1-1-benzyloxy-2-fluoroethyl ether, 2,2,2-trichloroethyl ether, 2-trimethylsilylethyl ether, 2-(phenylselenyl)ethyl ether, *t*-butyl ether, allyl ether, p-chlorophenyl ether, p-methoxyphenyl ether, 2,4-dinitrophenyl ether, benzyl ether, p-methoxylbenzyl ether, 3,4-dimethoxybenzyl ether, 2,6-dimethoxybenyzl, o-nitrobenzyl ether, p-nitrobenzyl ether, p-bromobenzyl ether, p-chlorobenzyl ether, 2,6-dichlorobenzyl ether, 2,4-dinitrobenzyl ether, fluorous benzyl ether, trimethylsilylxylyl ether, p-phenylbenzyl ether, cumyl ether, p-azidobenzyl ether, 2,6-difluorobenzyl ether, p-cyanobenzyl ether, p-phenylbenzyl ether, 2-picolyl ether, 4-picolyl ether, 3-methyl-2-picolyl *N-*oxido ether, diphenylmethyl ether, *p*,*p'*-dinitrobenzhydryl ether, 5-dibenzosuberyl ether, triphenylmethyl ether, α-naphtyldiphenylmethyl ether, p-methoxyphenyldiphenylmethyl ether, di(p-methoxyphenyl)phenylmethyl ether, tri(p-methoxyphenyl)phenylmethyl ether, 4-(4'-bromophenacyloxyphenyl)diphenylmethyl ether, 4,4',4"-tris(4,5-dichlorophthalimidophenyl)methyl ether, pentadienylnitrobenzyl, p-azidobenzyl ether, p-(methylsulfinyl)benzyl ether, 2-naphthylmethyl ether, 2-quinolinylmethyl ether, 1-pyrenylmethyl ether, 4-methoxydiphenylmethyl ether, 4-phenyldiphenylmethyl ether, α-naphthyldiphenylmethyl ether, p-methoxyphenyldiphenylmethyl ether, anthryl ether, 9-phenylthioxanthyl ether and the like; Silyl ethers such as trimethylsilyl ether, triethylsilyl ether, triisopropylsilyl ether, dimethylhexylsilyl ether, 2-norbomyldimethylsilyl ether, *t*-butyldimethylsilyl ether, *t-*butyldiphenylsilyl ether, tribenzylsilyl ether, tri-p-xylylsilyl ether, triphenylsilyl ether, diphenylmethylsilyl ether, di-*t*-butylmethylsilyl ether, bis(*t*-butyl)-1-pyrenylmethoxysilyl ether, tris(trimethylsilyl)silyl ether, (2-hidroxystyryl)dimethylsilyl ether, *t*-butoxydiphenylsilyl ether, 1,1,3,3-tetraisopropyl-3-[2-(tripheynlmethoxy)ethoxy]disiloxane-1-yl ether, fluorous silyl ether, and the like; Esters such as formate, benzoylformate, acetate, chloroacetate, dichloroacetate, trichloroacetate, trichloroacetimidate, trilfuoroacetate, methoxyacetate, triphenylmethoxyacetate, phenoxyacetate, p-chloropheynyacetate, phenylacetate, diphenylacetate, 3-phenylpropionate, bisfluorous chain type propanoyl ester, 4-pentenoate, levulinate, pivaloate, adamantoate, crotonate, 4-methoxylcrotonate, benzoate, p-phenylbenzoate, mesitoate, 4-bromobenzoate, 2,5-diflourobenzoate, p-nitrobenzoate, picolinate, nicotinate, 2-(azidomethyl)benzoate, 4-azidobutirate, (2-azidomethyl)phenylacetate, 2-{[(tritylthio)oxy]methyl}benzoate, 2-(allyloxy)phenylacetate, 2-(prenyloxymethyl)benzoate, 4-benzyloxybutyrate, 4-trialkylsiloxybutyrate, 4-acetoxy-2,2-dimethylbutyrate, 2,2-dimethyl-4-pentanoate, 2-iodobenzoate, 4-nitro-4-methylpentanoate, o-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloracetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, chlorodiphenylacetate, isobutyrate, monosuccionate, tigloate, o-(methoxycarbonyl)benzoate, p-benzoate, α-napthoate, nitrate, alkyl *N*,*N*,*N*',*N*'-tetramethylphosphorodiamidate, 2-chlorobenzoate, and the like; Sulfonates such as sulfate, allylsulfonate, methanesulfonate, benzylsulfonate, tosylate, 2-trifluoromethylsulfonate and the like; Carbonates such as alkyl methyl carbonate, methoxymethyl carbonate, 9-fluoromethyl carbonate, ethyl carbonate, bromoethyl carbonate, 2-(trimethylsilyl)ethyl carbonate, isobutyl carbonate, *t*-butyl carbonate, vinyl carbonate, allyl carbonate, propargyl carbonate, p-nitrophenyl carbonate, benzyl carbonate, 2-dansylethyl carbonate, phenacyl carbonate, methyl dithiocarbonate, S-benzyl thiocarbonate and the like; Carbamates such as dimethylthiocarbamate, N-phenylcarbamate, and the like; Cyclic acetals and ketals such as methylene acetal, ethylidene acetal, *t*-butylmethylidene acetal, 1-*t*-butylethylidine ketal, 1-phenyethylidene ketal, 2-(methoxycarbonyl)ethylidene acetal, 2-(*t*-butylcarbonyl)ethylidene acetal, phenylsulfonylethylidene acetal, 3-(benzyloxy)propylidene acetal, isopropylidene acetal or acetonide, cyclopentylidene acetal, benzylidene acetal, p-methoxybenzylidene acetal, mesitylene acetal, naphthaldehyde acetal, 9-anthracene acetal, benzophenone ketal and the like; Chiral ketones such as camphor ketal, menthone ketal and the like; Cyclic ortho esters such as methoxymethylene acetal, ethoxymethylene acetal, dimethoxymethylene orto ester, methylidene orto ester, phthalide orto ester, 1,2-dimethoxyethylidene orto ester, 2-oxacyclopentylidene orto ester, butane 2,3-bisacetal, cyclohexane-1,2-diacetal, dispiroketals and the like; Silyl derivatives such as di-*t*-butylsilylene group, diakkylsilylene group, 1,3-(1,1,3,3-tetraisopropyldisiloxanylidene) derivative, 1,1,3,3-tetra-*t-*butoxydisiloxanylidene derivative, methylene-bis-(diisopropylsilanoxanylidene, 1,1,4,4-tetrapheynyl-1,4-disilanylidene, o-xylyl ether, 3,3'-oxybis(dimethoxytrityl)ether, and the like; cyclic carbonates; cyclic borate such as methyl boronate, ethyl boronate, and the like.

The term "optionally substituted" when referring to alkyl, alkenyl, alkynyl, cycloalkyl, cycloalkenyl, aryl and heterocycle is intended to cover groups having oxo, ethylenedioxy, alkanoyloxy, alkoxy, alkylthio, carboxyl, halogen, thienyl, acetyl, 1-oxopropyl, 2-oxopropyl, 2-oxobutyl, 3-oxobutyl, 3-oxopentyl, 4-oxopentyl, 4-oxohexyl, 5-oxohexyl, ethylenedioxymethyl, 1,1-ethylenedioxyethyl, 2,2-ethylenedioxyethyl, 1,1-ethylenedioxypropyl, 2,2-ethylenedioxypropyl, 3,3-ethylenedioxypropyl, 1,1-ethylenedioxybutyl, 2,2-ethylenedioxybutyl, 3,3-ethylenedioxybutyl, 4,4-ethylenedioxybutyl, 3,3-ethylenedioxypentyl, 4,4-ethylenedioxyhexyl, 5,5-ethylenedioxyhexyl, acetyloxymethyl, 2-acetyloxyethyl, 3-acetyloxypropyl, 3-acetyloxybutyl, 4-acetyloxybutyl, 3-propionyloxybutyl, 3-butyryloxybutyl, 3-valeryloxypentyl, 3-hexanoyloxyhexyl, 4-acetyloxypentyl, 5-acetyloxypentyl, 4-acetyloxyhexyl, 5-acetyloxyhexyl, 6-acetyloxyhexyl, methoxymethyl, ethoxymethyl, propoxymethyl, butoxymethyl, pentyloxymethyl, hexyloxymethyl, 1-methoxyethyl, 2-methoxyethyl, 2-ethoxyethyl, 2-propoxyethyl, 2-butoxyethyl, 2-pentyloxyethyl, 2-hexyloxyethyl, 3-methoxypropyl, 3-ethoxypropyl, 2-methoxybutyl, 4-ethoxybutyl, 3-methoxypentyl, 5-ethoxypentyl, 4-methoxyhexyl, 6-ethoxyhexyl, methylthiomethyl, ethylthiomethyl, propylthiomethyl, butylthiomethyl, pentylthiomethyl, hexylthiomethyl, 1-methylthioethyl, 2-methylthioethyl, 2-ethylthioethyl, 2-methylthiopropyl, 3-methylthiopropyl, 3-ethylthiobutyl, 4-butylthiobutyl, 5-methylthiopentyl, 6-ethylthiohexyl, carboxymethyl, 1-carboxyethyl, 2-carboxyethyl, 2-carboxypropyl, 3-carboxypropyl, 4-carboxybutyl, 5-carboxypentyl, 6-carboxyhexyl, fluoromethyl, bromomethyl, chloromethyl, iodomethyl, 2-chloroethyl, 2-bromopropyl, 3-iodopropyl, 4-fluorobutyl, 5-chloropentyl, 6-bromohexyl, 2-thienylmethyl, 1-(2-thienyl)ethyl, 2-(2-thienyl)ethyl and the like.

The term "amino acid" refers to any of a class of organic compounds that contains at least one amino group, -NH-, and one carboxyl group, -COOH. These compounds can be the natural amino acids present in peptides or can contain any substitution in the amino group, in the carboxyl group or in the side chain. They can also present different chirality of the peptidic natural amino acids or can have different backbone, linear or cyclic, but must present, as said, at least one amino group and one carboxyl group. Amino acids can incorporate functional or protecting groups, such as those known for those skilled in the art (T.W.Greene, *vide supra*)*.* Preferred amino acids include, but are not limited to, alanine, valine, leucine and isoleucine.

Preferably, the organic solvent to be employed in the synthesis process is selected from methanol, ethanol, propanol, isopropanol, t-butanol, n-butanol, ethyl acetate, isopropyl acetate, butyl acetate, dichloromethane, toluene, tetrahydrofuran, 2-methyltetrahydrofuran, acetonitrile, acetone, cyclopentyl methyl ether, methyl ethyl ketone, methyl isobutyl ketone, dimethylamide, dimethylformamide and dimethylsulfoxide.

As mentioned above the compounds of formula (I) are useful as calcineurin inhibitors, preferably, for the prevention and/or treatment of a disease or disorder selected from an autoimmune disease, organ transplant rejection, cancer, heart-related disease or disorder, muscle-related disease or disorder, and neurological disease or disorder. Preferably, said autoimmune disease is selected from arthritis, anemia, psoriasis, dermatitis, urticaria, sclerosis, multiple sclerosis, inflammatory bowel disease, Crohn's disease, colitis, endocarditis, endometriosis, episcleritis, respiratory distress syndrome, meningitis, iritis, choroiditis, spondylitis, sudden hering loss, rhinitis, encephalitis, uveitis, autoimmune mocarditis, leukocyte adhesion deficiency, lupus, systemic lupus erythemotosus, diabetis mellitus, tuberculosis, sarcoidosis, granulomatosis, vasculitides, autoimmune aplastic anemia, autoimmune neutropenia, CNS inflammatory disorders, Bechet's or Behcet's disease, pemphigoid, pemphigus, Reiter's disease, nephritis, IgM polyneuropathies, thrombocytopenia, hypothyroidism, hypoparathyroidism, thyroiditis, Grave's disease, polyglandular syndromes, paraneoplastic syndromes, encephalomyelitis, myasthenia gravis, autoimmune hepatitis, Guillain-Barre syndrome, Berger's disease, dermatosis, Celiac disease, amylotrophic lateral sclerosis, coronary artery disease, autoimmune hearing loss, polychondritis, paraneoplasic syndrome, channelopathies, epilepsy, migraine, autism, fibromyalgia, multiple endocrine failure, presenile dementia, Chagas' disease, rheumatic fever, recurrent abortion, erythema multiforme, alveolitis, leprosy, malaria, leishmaniasis, dengue, endocarditis, fibrosis, cyclitis, human immunodeficiency virus (HIV) infection, Alzheimer's disease, Epstein-Barr virus infection, keratoconjuctivitis, bronchitis, chronic obstructive airway disease, idiophatic facial paralysis, chronic fatigue syndrome, febris rheumatica, hypogonadism, pancreatitis, vitiligo, acquire immune deficiency syndrome, multiple organ injury syndrome, autoimmune atrophic gastritis, rheumatic diseases, myocarditis, nephrotic syndrome, sinusitis, eosinophilia, hyperalgesia, meningitis, diabetic disorders, valvulitis, Addison's disease, juvenile dermatomyositis, scleroderma, periorificial dermatitis, steroid-resistant nephrotic syndrome and Focal segmental glomerulosclerosis (FSGS). More preferably, said autoimmune disease is selected from arthritis, anemia, psoriasis, dermatitis, sclerosis, multiple sclerosis, meningitis, rhinitis, lupus, diabetis mellitus, CNS inflammatory disorders, pemphigus, nephritis, autoimmune hepatitis, Celiac disease, migraine, fibromyalgia, endocarditis, coronary artery disease, epilepsy, Chagas' disease, rheumatic fever, malaria, Alzheimer's disease, Epstein-Barr virus infection, bronchitis, chronic obstructive airway disease, chronic fatigue syndrome, pancreatitis, acquire immune deficiency syndrome, multiple organ injury syndrome, myocarditis, meningitis, diabetic disorders, Addison's disease, juvenile dermatomyositis, scleroderma, periorificial dermatitis, steroid-resistant nephrotic syndrome and Focal segmental glomerulosclerosis (FSGS).

In a particular embodiment, the compounds of formula (I) are useful as calcineurin inhibitors, preferably, for the prevention and/or treatment of cancer. Examples of cancer include, but they are not limited to, sarcoma, blastoma, neurological cancer, Hedgehog pathway related cancer, rhabdomyosarcoma, neuroblastoma, medulloblastoma, intracranial cancers, brain cancers, including brain metastases, glioma, glioblastoma, pediatric brain tumors, embryonal tumors, pineoblastoma, oligodendroglioma, pituitary tumors, Ewing's sarcoma, meningioma, ependymoma, craniopharyngioma, choroid plexus carcinoma, astrocytoma, acoustic neuroma, and soft tissues sarcoma.

As mentioned above, calcineurin (CN or PPP3, formerly PP2B) is a serine/threonine protein phosphatase regulated by Ca2+ and calmodulin. CN regulates development, memory, cardiac function, and immune response by dephosphorylating a variety of protein substrates, including the family of cytosolic Nuclear Factor of Activated T-cells (NFATc) transcription factors. NFATc proteins have been shown to be direct substrates of calcineurin. Dephosphorylated NFATc proteins translocate into the nucleus where, in cooperation with other transcription factors, induce the expression of many cytokine and chemokine genes, leading to T cell activation. It also plays a prominent role in many physiological processes, including homeostasis, angiogenesis, myogenesis, adipogenesis, osteogenesis, chondrocyte differentiation, cardiovascular system development, pancreatic β-cell proliferation, hair follicle cell differentiation and remodelling, and activities of cells of the immune and nervous systems, including schizophrenia, diabetes mellitus, Down synthdrome, cardiac hypertrophy, as well as in neurodegenerative diseases, such as Alzheimer's disease, psychotic disorders, epilepsy, among others.

Compounds of formula (I) are have surprisingly found to be inhibitors of the calciurenin-NFAT signaling pathway. NFAT proteins are expressed in immune cells and play a key role in eliciting immune responses. The NFAT proteins are activated by calcineurin, and the activated NFAT proteins, in turn, induce transcription of cytokine genes which are required for an immune response. By NFAT or NFATc protein (nuclear factor of activated T cells) is meant a member of a family of transcription factors comprising the members NFAT1, NFAT2, NFAT3 and NFAT4, with several isoforms. Any other NFATc protein whose activation is calcineurin dependent is also meant to be included herien. NFATc proteins can be, e.g., mammalian proteins, e.g., human or murine. NFAT1, NFAT2 and NFAT4 are expressed in immune cells, e.g., T lymphocytes, and play a role in eliciting immune responses. NFATc proteins are involved in the transcriptional regulation of cytokine genes, e.g., IL-2, IL-3, IL-4, TNF-α and IFN-γ, during the immune response.

This action as inhibitors of the calciurenin-NFAT signaling pathway renders the compounds of formula (I) also useful for inhibiting or preventing activity of the immune system, being able these compounds of formula (I) to act as an immunosuppresive drug.

Accordingly, the present invention relates to an immunosuppressive agent (compound of formula (I)) that inhibits interaction between calcineurin and NFAT with reduced toxic effects, by selectively inhibiting the interaction between calcineurin and NFAT, without inhibiting the enzymatic activity of calcineurin for its other substrates. Accordingly, these immunosuppressive agent exhibits reduced side effects in comparison to the gold standard treatments using cyclosporine A or tacrolimus.

Compounds of formula (I) do not disrupt CN-NFATc interaction by the PxlxlT motif, although compounds of formula (I) strongly inhibit NFTAc activation in human HEK 293T cells at several concentrations, *i.e.* compounds of formula (I) behave as potent calcineurin inhibitors useful as immunosuppressive drugs.

Furthermore, compounds of formula (I) do not affect the phosphatase activity of CNA towards pNPP or RII substrates, i.e. compounds of formula (I) avoid the severe side effects associated to current immunosuppressive drugs such as cyclosporine or tacrolimus that bind to calcineurin through its catalytic site.

To sum up, compounds of formula (I) exhibit a more efficient and selective activity as calcineurin inhibitors than current cornerstone drugs such as cyclosporine and tacrolimus.

In a second aspect, the present invention relates to a compound of formula I as defined above, including stereoisomers and pharmaceutically acceptable salts thereof, wherein:
R₁ is: 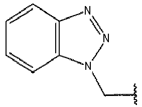
R₂ is: 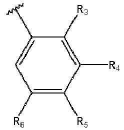 wherein:
   R₃, R₄, and R₆ are independently H, alkyl, halo or carboxyl;
   R₅ is H, halo, or cyano.

In a preferred embodiment, the compound of formula (I) is a compound wherein:
R₃, R₄, and R₆ are independently H, C₁₋₃alkyl, halo, or carboxyl;
R₅ is H or halo; and
wherein at least two of R₃, R₄, R₅ and R₆ are different from hydrogen.

In a preferred embodiment, the compound of formula (I) is a compound wherein:
R₃ is methyl;
R₄ is H, F, or CI;
R₅ is H or F;
R₆ is H, F, or carboxyl;
wherein at least two of R₄, R₅ and R₆ are different from hydrogen; and
at least one R₄, R₅ and R₆ is halogen.

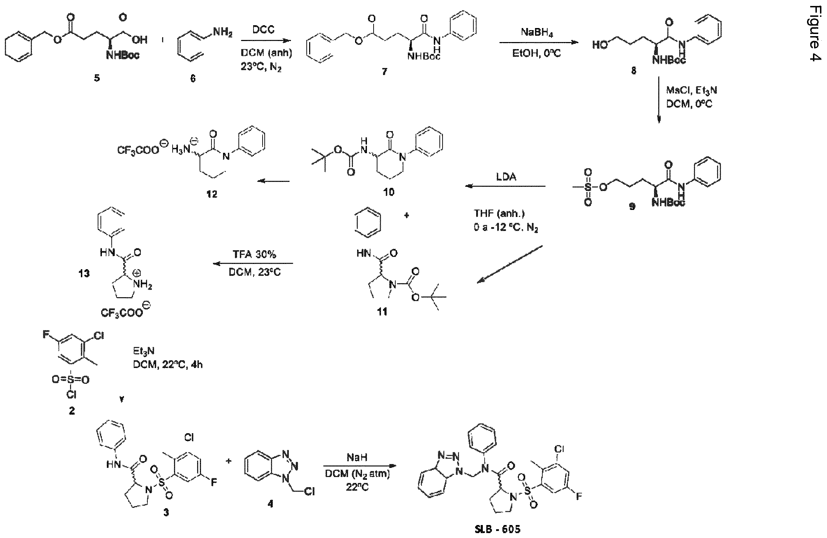

These new compounds are obtained according to usual chemical synthesis found in the literature. An example of a synthesis scheme is shown in figure 4, wherein the compound SLB-605 is obtained.

### EXAMPLES

### Example 1A. Synthesis of racemic INK-150.46 (also named SLB-0605)

| **Compound internal code** | **R₁** | **R₂** |
|---|---|---|
| INK-150.46 | 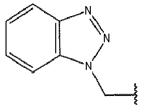 | 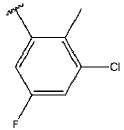 |

### Step a) tert-Butyl 2-(phenylcarbamoyl)pyrrolidine-1-carboxylate (rac-11)

To a stirred solution of racemic Boc-proline (207 mg, 0.962 mmol) in anhydrous CH₂Cl₂ (5 mL) at room temperature, 1-[bis(dimethylamino)methylene]-1*H*-1,2,3-triazolo[4,5-b]pyridinium 3-oxid hexafluorophosphate (HATU, 439 mg, 1.15 mmol, 1.2 eq) was added. After 10 minutes, aniline (0.13 mL, 1.44 mmol, 1.5 eq) and triethylamine (0.27 mL, 1.92 mmol, 2 eq) were added dropwise and the reaction mixture was stirred at room temperature until total consumption of starting materials (16 h). The mixture was evaporated to dryness and chromatographically purified over SiO₂ using Hexane/EtOAc 93/7, to provide compound **rac-11** (275 mg, 100% purity, 98% yield).

¹H NMR (400 MHz, CDCl₃) δ 9.46 (s, 1H), 7.52 (d, J = 7.6 Hz, 2H), 7.31 (t, J = 7.6 Hz, 2H), 7.09 (t, J = 6.5 Hz, 1H), 4.56 - 4.20 (m, 1H), 3.64 - 3.22 (m, 2H), 2.72 - 2.09 (m, 1H), 2.08 - 1.75 (m, 3H), 1.49 (s, 9H).

HPLC-MS Ret (Method B): 1.79 min; ESI+-MS m/z: 191 (M-Boc)+.

Chiral HPLC: The two enantiomers were successfully separated under chiral HPLC conditions. Ret (Method D bis, Column Chiralpak IC): 6.44 min & 9.16 min.

### Step b) N-Phenylpyrrolidine-2-carboxamide (rac-13)

To a stirred solution of **rac-11** (275 mg, 0.947 mmol) in 6 mL of CH₂Cl₂, trifluoroacetic acid mL, 21.8 mmol, 23 eq) was added at room temperature, and the mixture was sealed and stirred for 0.5 h. After then, the crude was evaporated to dryness, yielding an orange oily residue of the organic salt **rac-13,** which was used without further purification (quantitative yield).

¹H NMR (400 MHz, CDCl₃) δ 11.26 (s, 1H), 9.97 (s, 1H), 7.52 (d, J = 7.6 Hz, 2H), 7.31 - 7.26 (m, 2H), 7.50 - 7.26 (m, 1H), 7.12 (t, *J* = 7.4 Hz, 1H), 5.14 - 4.74 (m, 1H), 3.58 - 3.39 (m, 2H), 2.53 (dq, *J* = 7.0, 14.3 Hz, 1H), 2.15 (dq, *J* = 6.7, 13.0 Hz, 1H), 2.09 - 1.98 (m, 2H). HPLC-MS Ret (Method B): 1.23 min; ESI+-MS m/z: 191 (M-Boc)+.

Chiral HPLC: The two enantiomers were successfully separated under chiral HPLC conditions. Ret (Method C bis, Column Chiralpak IC): 6.74 min & 8.82 min.

### Step c) 1-((3-Chloro-5-fluoro-2-methylphenyl)sulfonyl)-N-phenylpyrrolidine-2-carboxamide (rac-3)

To a stirred solution of **rac-13** (288 mg, 0.947 mmol) in anhydrous DMF (4 mL), anhydrous Et₃N (0.46 mL, 3.31 mmol, 3.5 eq) was added at 0 °C. After 10 min, compound 2 (see figure 4) was added (345 mg, 1.42 mmol, 1.5 eq) at the same temperature. The mixture was stirred for 2 h at 0 °C and then, it was evaporated to dryness. It was purified over silica gel using Hexane/EtOAc from 95/5 to 85/15 as the eluant, yielding the desired product **rac-3** as a white solid (350 mg, 100% purity, 93% yield).

¹H NMR (400 MHz, CDCl₃) δ 8.60 (s, 1H), 7.79 (d, J = 7.4 Hz, 1H), 7.61 - 7.53 (m, 2H), 7.40 - 7.27 (m, 3H), 7.19 - 7.09 (m, 1H), 4.47 - 4.31 (m, 1H), 3.66 - 3.54 (m, 1H), 3.50 - 3.36 (m, 1H), 2.54 - 2.43 (m, 1H), 2.39 (s, 3H), 2.01 - 1.78 (m, 3H).

HPLC-MS Ret (Method B): 2.19 min; ESI+-MS m/z: 397 (M+H)+.

Chiral HPLC: The two enantiomers were successfully separated under chiral HPLC conditions. Ret (Method C bis, Column Chiralpak IC): 6.97 min & 7.67 min.

### Step d) N-((1H-Benzo[d][1,2,3]triazol-1-yl)methyl)-1-((3-chloro-5-fluoro-2-methylphenyl)sulfonyl)-N-phenylpyrrolidine-2-carboxamide (rac-SLB-0605)

To a stirred suspension of sodium hydride (60% purity, 106 mg, 2.65 mmol, 3 eq) in anhydrous DMF (2 mL), at 0 °C, a solution of **rac-3** (350 mg, 0.882 mmol) in anhydrous DMF (5 mL), was added. After 1 h stirring at the same temperature, a solution of compound 4 (see figure 4) (443 mg, 2.65 mmol, 3 eq) in anhydrous DMF (4.5 mL) was added and allowed to reach room temperature overnight. After 16 h, it was evaporated to dryness and purified over silica gel using Hexane/EtOAc from 95/05 to 70/30 as the eluant, yielding the desired product **rac-SLB-0605** as a white solid (387 mg, 100% purity, 83% yield).

¹H NMR (400 MHz, CDCI3) δ 8.05 (dt, J = 0.9, 8.4 Hz, 1H), 7.91 (dt, J = 0.9, 8.4 Hz, 1H), 7.63 (d, J = 7.4 Hz, 1H), 7.53 (ddd, *J* = 1.0, 7.0, 8.3 Hz, 1H), 7.45 - 7.31 (m, 4H), 7.04 (d, J = 9.7 Hz, 1H), 7.12-6.75 (m, 2H), 6.62 (d, *J* = 13.8 Hz, 1H), 6.37 (d, *J* = 13.8 Hz, 1H), 4.34 (dd, J = 4.9, 7.9 Hz, 1H), 3.75 - 3.60 (m, 1H), 3.45 - 3.31 (m, 1H), 2.33 (s, 3H), 2.17 - 2.01 (m, 1H), 1.88 - 1.75 (m, 2H), 1.75 - 1.64 (m, 1H).

HPLC-MS Ret (Method B): 2.33 min; ESI+-MS m/z: 528 (M+H)+.

Chiral HPLC: The two enantiomers were successfully separated under chiral HPLC conditions. Ret (Method D, Column Chiralpak IA): 11.05 min & 14.08 min.

### Example 1B. Synthesis of INK-150.46 (also named SLB-0605) - S enantiomer

### Step a) Benzyl (S)-4-((tert-butoxycarbonyl)amino)-5-oxo-5-(phenylamino)pentanoate (7, see figure 4)

To a stirred solution of Boc-*L*-glutamic acid 5-benzyl ester (5 (see figure 4), 1.007 g, 2.985 mmol) in anhydrous CH₂Cl₂ (15 mL) at 0 °C, dicyclohexylcarbodiimide (DCC, 739 mg, 3.582 mmol) was added. After 15 minutes, aniline (0.28 mL, 3.10 mmol) dissolved in anhydrous CH₂Cl₂ (5 mL) was added dropwise to the reaction mixture over 10 minutes at 0 °C. The orange suspension was stirred for 15 minutes at 0 °C and at rt until total consumption of starting materials (18 h). The mixture was filtered to remove insoluble material and the resulting filtrate was evaporated to dryness and chromatographically purified over SiO₂ using Hexane/EtOAc 85/15, to provide compound 7 (see figure 4) (1.231 g, 100% purity, 100% yield).

¹H NMR (400 MHz, CDCl₃) δ 8.35 (s, 1H), 7.50 (dd, J = 1.0, 8.6 Hz, 2H), 7.40 - 7.27 (m, 7H), 7.11 (t, *J* = 7.4 Hz, 1H), 5.33 (d, *J* = 7.5 Hz, 1H), 5.15 (d, J = 2.5 Hz, 2H), 4.38 - 4.17 (m, 1H), 2.65 (ddd, J = 6.4, 7.7, 16.8 Hz, 1H), 2.51 (dt, J = 6.7, 17.0 Hz, 1H), 2.33 - 2.15 (m, 1H), 2.01 (dq, *J* = 6.8, 14.4 Hz, 1H), 1.45 (s, 9H).

HPLC-MS Ret (Method B): 2.25 min; ESI+-MS m/z: 435 (M+Na)+.

Chiral HPLC: The compound was submitted to different chiral HPLC conditions and in all cases only one peak was observed. Ret (Method C, Column Chiralpak AS-H): 5.86 min.

### Step b) tert-Butyl (S)-(5-hydroxy-1-oxo-1-(phenylamino)pentan-2-yl)carbamate (8, see figure 4)

To a stirred suspension of NaBH₄ (448 mg, 11.83 mmol) in EtOH (0.14 L) at 0 °C crushed CaCl₂ (689 mg, 6.21 mmol) was carefully added during 1 min. Then, compound 7 (see figure 4) (1.220 g, 2.958 mmol) dissolved in EtOH (20 + 5 + 5 mL) was added in portions during 10 min. The solution was stirred for 3.5 h allowing it to warm to rt. The crude product was neutralized at 0 °C using NH₄Cl aqueous saturated solution, and the aqueous phase was extracted with EtOAc. The organic phase was washed with NaCl aqueous saturated solution, dried over anhydrous Na₂SO₄ and evaporated to dryness. The resulting oily residue was chromatographically purified over SiO₂ using Hexane/EtOAc (35:65), to give compound **8** (See figure 4) (909 mg, 100%).

¹H NMR (400 MHz, CDCl₃) δ 8.67 (s, 1H), 7.51 (dd, J = 1.1, 8.6 Hz, 2H), 7.37 - 7.27 (m, 2H), 7.13 - 7.05 (m, 1H), 5.42 (d, J = 7.2 Hz, 1H), 4.49 - 4.29 (m, 1H), 3.87 - 3.70 (m, 2H), 2.42 (s, 1H), 1.99 (dt, *J* = 6.9, 13.7 Hz, 1H), 1.84 (dq, *J* = 7.4, 13.9 Hz, 1H), 1.74 - 1.63 (m, 2H), 1.45 (s, 9H).

HPLC-MS Ret (Method B): 1.52 min; ESI+-MS m/z: 331 (M+Na)+.

Chiral HPLC: The compound was submitted to different chiral HPLC conditions and in all cases only one peak was observed. Ret (Method D, Column Chiralpak IB): 5.34 min.

### Step c) (S)-4-((tert-Butoxycarbonyl)amino)-5-oxo-5-(phenylamino)pentyl methanesulfonate (9, see figure 4)

To a stirred solution of compound **8** (see figure 4) (0.898 g, 2.912 mmol) in anhydrous CH₂Cl₂ (30 mL) at 0 °C, anhydrous Et3N (0.61 mL, 4.37 mmol, 1.5 eq) was added. After 10 min, methanesulfonyl chloride (0.27 mL, 3.49 mmol, 1.2 eq) was added dropwise at 0 °C. The mixture was stirred for 10 min at 0 °C and at rt for 2.5 h. Then, the crude product was chromatographically purified over SiO₂ using Hexane/EtOAc (50:50), to give 9 (see figure 4) (1.111 g, 99% yield) that was used in the following step as such.

1H NMR (400 MHz, CDCl₃) δ 8.23 (s, 1H), 7.52 (dd, J = 1.1, 8.6 Hz, 2H), 7.37 - 7.28 (m, 2H), 7.11 (t, *J* = 7.4 Hz, 1H), 5.21 (d, J = 5.5 Hz, 1H), 4.56 - 4.42 (m, 1H), 4.41 - 4.26 (m, 1H), 4.31 (dt, J = 5.1, 10.3 Hz, 1H), 3.05 (s, 3H), 2.16 - 2.05 (m, 1H), 2.00 - 1.84 (m, 2H), 1.84 - 1.71 (m, 1H), 1.46 (s, 9H).

HPLC-MS Ret (Method B): 1.80 min; ESI+-MS m/z: 409 (M+Na)+.

Chiral HPLC: The compound was submitted to different chiral HPLC conditions and in all cases only one peak was observed. Ret (Method D, Column Chiralpak AD-H): 18.37 min.

### Step d) (S)-tert-Butyl 2-(phenylcarbamoyl)pyrrolidine-1-carboxylate (11, see figure 4)

To a stirred solution of 9 (see figure 4) (1.109 g, 2.87 mmol) in anhydrous tetrahydrofuran (24 mL), at 50 °C under argon atmosphere, potassium tert-butoxyde (0.34 g, 3.01 mmol, 1.05 eq) was added, and the mixture was kept stirring at 50 °C during 1 h. The reaction crude was evaporated to dryness and the residue was chromatographically purified over SiO₂ using Hexane/EtOAc (85:15), to give 11 (See figure 4) (405 mg, 49% yield).

¹H NMR (400 MHz, CDCl₃) δ 7.56 (d, J = 7.7 Hz, 2H), 7.32 (t, J = 7.5 Hz, 2H), 7.10 (t, J = 6.8 Hz, 1H), 4.47 - 4.20 (m, 1H), 3.71 - 3.38 (m, 2H), 2.41 - 2.17 (m, 1H), 2.03 (dq, J = 5.8, 6.3, 11.2 Hz, 2H), 1.97 - 1.77 (m, 1H), 1.37 (s, 9H).

HPLC-MS Ret (Method B): 1.79 min; ESI+-MS m/z: 313 (M+Na)+.

Chiral HPLC: The compound was submitted to the same chiral HPLC conditions used in the racemic route for this intermediate and peaks corresponding to the two enantiomers were observed in a ratio 94.6:5.4 (89.2% ee).

Ret (Method D bis, Column Chiralpak IC): 6.45 & 9.20 min, ratio 94.6:5.4 (89.2% ee).

When increasing polarity in the chromatography (until 25-40% of EtOAc) the 6-membered isomer (10, see figure 4) was also isolated (230 mg, 28% yield). The separation was good although in the standard HPLC conditions (Method B) both compounds, 10 and 11 (see figure 4), elute very closely.

¹H NMR (400 MHz, CDCl₃) δ 7.43 - 7.35 (m, 2H), 7.29 - 7.20 (m, 3H), 5.54 (s, 1H), 4.26 (dt, *J* = 5.6, 11.3 Hz, 1H), 3.70 (td, *J* = 1.7, 6.4 Hz, 2H), 2.61 (dq, *J* = 5.3, 11.3 Hz, 1H), 2.16 - 1.98 (m, 2H), 1.71 (tt, *J* = 8.4, 12.3 Hz, 1H), 1.46 (s, 9H).

HPLC-MS Ret (Method B): 1.75 min; ESI+-MS m/z: 291 (M+H)+.

### Step e) (S)-N-Phenylpyrrolidine-2-carboxamide (trifluoroacetate salt, 13, see figure 4)

To a stirred solution of **11** (see figure 4) (395 mg, 1.36 mmol) in CH₂Cl₂ (10 mL), trifluoroacetic acid (2.4 mL, 31.29 mmol, 23 eq) was added at room temperature, and the mixture was sealed and stirred for 1 h. The crude was evaporated to dryness, yielding an orange oily residue of the organic salt 13 (see figure 4), which was used without further purification (quantitative yield).

¹H NMR (400 MHz, CDCl₃) δ 11.54 (s, 1H), 10.06 (s, 1H), 7.48 (dd, *J* = 1.1, 8.6 Hz, 2H), 7.45 - 7.29 (m, 1H), 7.30 - 7.24 (m, 2H), 7.18 - 7.02 (m, 1H), 4.87 (t, J = 7.5 Hz, 1H), 3.49 - 3.30 (m, 2H), 2.60 - 2.43 (m, 1H), 2.14 (td, J = 6.7, 13.0, 13.6 Hz, 1H), 2.08 - 1.93 (m, 2H).

HPLC-MS Ret (Method B): 1.23 min; ESI+-MS m/z: 191 (M+H)+.

Chiral HPLC: The compound was submitted to the same chiral HPLC conditions used in the racemic route for this intermediate and peaks corresponding to the two enantiomers were observed in a ratio 94.1:5.9, (88.2% ee).

Ret (Method C bis, Column Chiralpak IC): 6.74 & 8.83 min, ratio 94.1:5.9 (88.2% ee).

### Step f) (S)-1-((3-Chloro-5-fluoro-2-methylphenyl)sulfonyl)-N-phenylpyrrolidine-2-carboxamide (3, see figure 4)

To a stirred solution of **13** (see figure 4) (389.8 g, 1.28 mmol) in anhydrous DMF (5 mL), anhydrous Et₃N (0.62 mL, 4.48 mmol, 3.5 eq) was added at 0 °C. After 10 min, compound **2** (see figure 4) was added (467 mg, 1.92 mmol, 1.5 eq) at 0 °C and the mixture was stirred for 2 h at the same temperature. Then, the solvent was removed under vacuum and the residue was purified over silica gel using Hexane/EtOAc from 95/5 to 85/15 as the eluant, yielding the desired product 3 (see figure 4) as a white solid (508 mg, 100% purity, 100% yield).

¹H NMR (400 MHz, CDCl₃) δ 8.60 (s, 1H), 7.79 (dd, J = 3.5, 7.3 Hz, 1H), 7.64 - 7.53 (m, 2H), 7.40 - 7.27 (m, 3H), 7.18 - 7.09 (m, 1H), 4.44 - 4.33 (m, 1H), 3.65 - 3.52 (m, 1H), 3.48 - 3.37 (m, 1H), 2.53 - 2.42 (m, 1H), 2.39 (s, 3H), 2.05 - 1.79 (m, 3H).

HPLC-MS Ret (Method B): 2.19 min; ESI+-MS m/z: 397 (M+H)+.

Chiral HPLC: The compound was submitted to the same chiral HPLC conditions used in the racemic route for this intermediate and peaks corresponding to the two enantiomers were observed in a ratio 5.9:94.1 (88.2% ee).

Ret (Method C bis, Column Chiralpak IC): 6.98 & 7.98 min, ratio 5.9: 94.1 (88.2% ee).

### Step g) (S)-N-((1H-Benzo[d][1,2,3]triazol-1-yl)methyl)-1-((3-chloro-5-fluoro-2-methylphenyl)sulfonyl)-N-phenylpyrrolidine-2-carboxamide (SLB-0605)

To a stirred solution of sodium hydride (154 mg, 3.84 mmol) in anhydrous DMF (2.5 mL) at 0 °C under argon atmosphere, a solution of 3 (See figure 4) (508 mg, 1.28 mmol) in anhydrous DMF (7 mL) was added. After 1 h at 0 °C, 1-(chloromethyl)-1H-benzotriazole (644 mg, 3.84 mmol, 3 eq) in anhydrous DMF (5 mL) was added and then the reaction mixture was allowed to reach room temperature and stirred during 18 h. Then, the solvent was removed under vacuum and it was purified over silica gel using Hexane/EtOAc from 95/5 to 75/25 as the eluant, yielding the desired product **SLB-0605** as a white solid (604 mg, 100% purity, 89% yield).

¹H NMR (400 MHz, CDCl₃) δ 8.05 (dt, J = 0.9, 8.4 Hz, 1H), 7.91 (dt, J = 0.8, 8.4 Hz, 1H), 7.63 (d, J = 7.5 Hz, 1H), 7.53 (ddd, *J* = 1.0, 7.0, 8.2 Hz, 1H), 7.47 - 7.32 (m, 4H), 7.04 (d, J = 9.7 Hz, 1H), 7.11 - 6.77 (m, 2H), 6.62 (d, *J* = 13.8 Hz, 1H), 6.37 (d, *J* = 13.8 Hz, 1H), 4.35 (dd, J = 4.8, 7.8 Hz, 1H), 3.67 (dt, J = 7.1, 9.4 Hz, 1H), 3.38 (dt, J = 6.7, 9.3 Hz, 1H), 2.33 (s, 3H), 2.18 - 2.05 (m, 1H), 1.84 - 1.74 (m, 2H), 1.69 (dt, *J* = 6.6, 12.3 Hz, 1H).

HPLC-MS Ret (Method B): 2.33 min; ESI+-MS m/z: 528 (M+H)+.

Chiral HPLC: The compound was submitted to the same chiral HPLC conditions used in the racemic route for this final compound and 2 peaks were observed in a ratio 5.9:94.1 (88.2% ee).

Ret (Method D, Column Chiralpak IA): 11.07 & 14.10 min, ratio 5.9:94.1 (88.2% ee).

### Analytical methods:

METHOD A (not employed): Column ZORBAX Extend-C18 RRHD 2.1 x 50 mm, 1.8 µm, temperature 35 °C; flow rate 0.61 mL/min; A: NH4HCO3 10 mM, B: MeCN; gradient 0.3 min 98% A, 98% A to 100% B in 2.65 min; isocratic 2.05 min 100% B.
METHOD B: Column ZORBAX Extend-C18 RRHD 2.1 x 50 mm, 1.8 µm, temperature 35 °C; flow rate 0.61 mL/min; A: NH4HCO3 10 mM, B: MeCN, C: MeOH + 0.1% formic acid; gradient 0.3 min 98% A, 98%A to 0:95:5 A:B:C in 2.7 min; 0:95:5 A:B:C to 100% B in 0.1 min; isocratic 2 min 100% B.
METHOD C (not employed): Column ZORBAX Extend-C18 RRHD 4.6mm x 250mm, 5µm, temperature: r.t.; eluent n-Heptane/EtOH/ DEA 50/50/0.1 v/v/v, flow rate: 0.8ml/min.
METHOD C bis: Column ZORBAX Extend-C18 RRHD 4.6mm x 250mm, 5µm, temperature: r.t.; eluent n-Heptane/EtOH/ DEA 70/30/0.1 v/v/v, flow rate: 0.8ml/min.
METHOD D: Column ZORBAX Extend-C18 RRHD 4.6mm x 250mm, 5µm, temperature: r.t.; eluent n-Heptane/IPA/ DEA 50/50/0.1 v/v/v, flow rate: 0.8ml/min.
METHOD D bis: Column ZORBAX Extend-C18 RRHD 4.6mm x 250mm, 5µm, temperature: r.t.; eluent n-Heptane/IPA/ DEA 70/30/0.1 v/v/v, flow rate: 0.8ml/min.

### Example 2. Compounds of formula (I) inhibit NFATc activation in HEK 293T cells

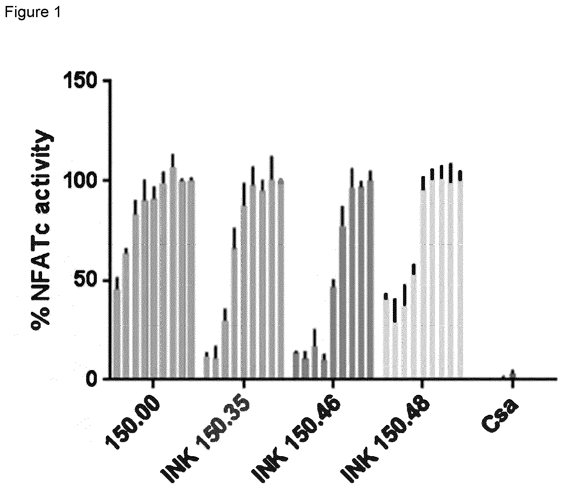

To determine the effect of compounds of formula (I) on NFATc activation, HEK 293T cells were transfected with 3xNFAT-luc reporter gene and Renilla-luc plasmid as control of gene transfection. After 24 hours, to activate the cellular CN -NFATc signaling pathway, cells were stimulated with ionomycin (lo), PMA and CaCl₂ for 4 h to raise intracellular concentration of calcium. As positive control of inhibition of CN activity, cells were treated with 1 µM of CsA 30 min prior to Io/PMA/CaCl2 stimulation. Then, cells were treated with compounds of formula (I) at several concentrations for 2 hours and light units analyzed following manufacture's protocol of the dual-luciferase reporter assay system (Figure 1, Functional effect of pair related structure compounds on NFATc activation).

Results from Figure 1 show that INK-150.35, INK-150.46 and INK-150.48 compounds strongly inhibits NFATc activation in HEK 293T cells. This inhibition takes place at least at 100, 50 and 20 µM of the concentration for the compounds.

| **Compound internal code** | **R₁** | **R₂** |
|---|---|---|
| INK-150.00 | 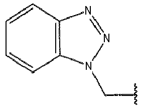 | 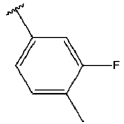 |
| INK-150.35 | 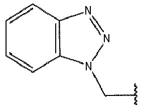 | 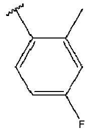 |
| INK-150.46 | 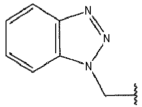 | 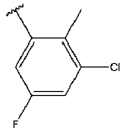 |
| INK-150.48 | 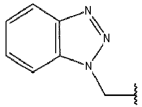 | 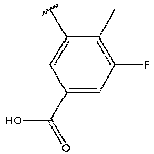 |

### Example 3. Compounds of formula (I) do not inhibit calcineurin phosphatase activity towards the pNPP substrate

CN dephosphorylation activity is commonly evaluated towards the small molecule pNPP, to determine the accessibility of small molecules to the active site of CN enzyme, or towards the RII peptide, from the regulatory RII subunit of cAMP-dependent protein kinase, as a big substrate that binds to CNA prior dephosphorylation by the CN active site.

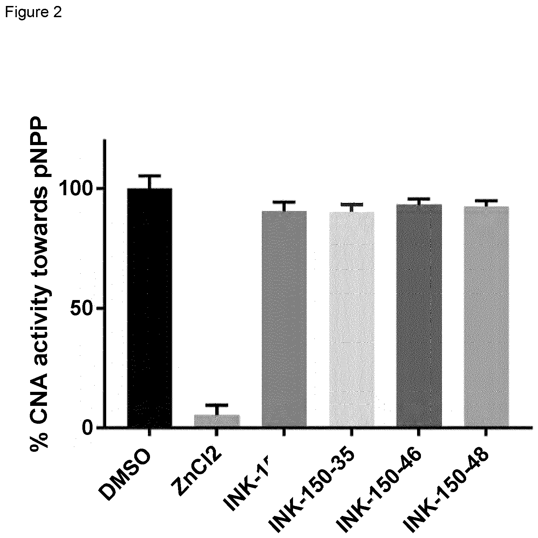

To determine the functional effect of compounds of formula (I) on CN phosphatase activity towards the pNPP substrate, a biochemical assay was performed. The assay evaluates the ability of recombinant human CNA (amino acids 2-347) fused to glutathione S-transferase (GST-CN) to dephosphorylate the pNPP substrate. First, GST-CN was expressed at high amounts in bacteria, purified and then CN activity analyzed, in the absence of any compound, towards the pNPP substrate. Then, 50 µM of each compound was analyzed to determine their inhibitory potential towards the phosphatase activity of CN (Figure 2).

### Example 4. Compounds of formula (I) do not inhibit calcineurin phosphatase activity towards the RII substrate

Compound INK-150.00 that inhibit NFATc activity was also analyzed for their ability to inhibit CN full length protein towards the RII substrate by using the CN phosphatase assay kit (Enzo Life Sciences) and following manufacturer's instructions.

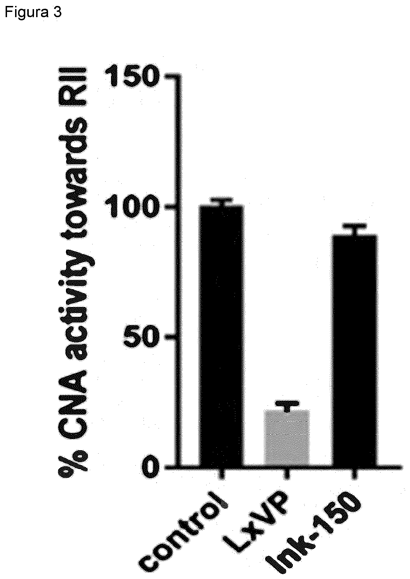

Compounds of formula (I) do not seem to affect phosphatase activity of CN towards the RII substrate (figure 3).

In conclusion, compounds of formula (I) do not affect the phosphatase activity of CN towards pNPP or RII substrates. Compounds of formula (I) show a strong inhibitory effect of NFATc activation in human 293T cells at several concentrations.

However, compounds of formula (I) compete with NFATc binding to CN by a site different from the previously described PxIxIT- and LxVP-binding motifs on CAN.

### Example 5. Assay of compound SLB-605 against several cancer cell lines

Compound INK-150.46 (or SLB-605) was assayed against several cancer cell lines in proliferation assays.

The measurements were taken using an *in vitro* test to measure cell growth based on Methyl violet staining after 5 days of incubation with the aforementioned drug or control vehicle (DMSO). The procedure was carried out in a 96-well plate. After 1 wash with phosphate-buffered saline (PBS), cells were stained for 20 minutes at room temperature with Methyl violet (0.5 % w/v in water). After 5 washes with PBS, and the addition of acetic acid (10% in water), plates were read in a spectrophotometer at 590 nm. All conditions were tested in 6 independent wells and results expressed as a mean of all replicates.

As shown, compound SLB-605 showed good inhibition of cellular proliferation in assayed lines.

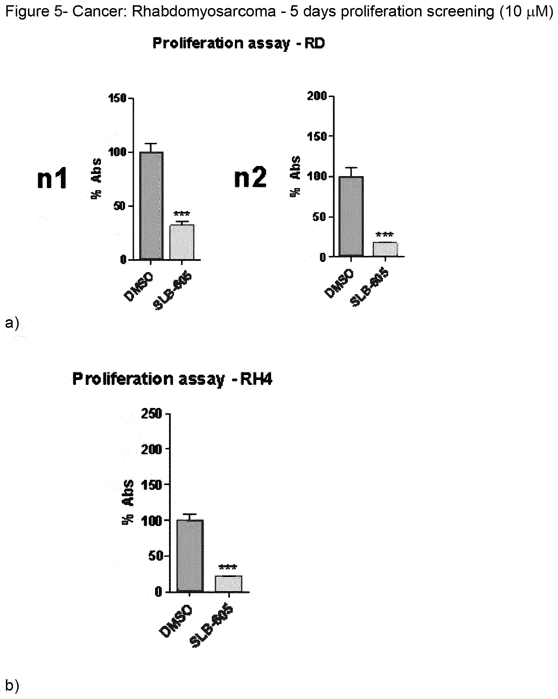

See figures 5, 6, 7 and the table below:

| Compound | Effect over RD (%) | Effect over RH4 (%) | Effect over SKNBE2 (%) | Effect over SHSY5Y (%) |
|---|---|---|---|---|
| SLB-605 | 75 | 78.1 | 59.5 | 48.8 |

The upper table shows the inhibitory effect of compound over several cancer cell lines. Results are expressed as inhibition values (%), thus the higher value the better result.

Figure 5 graphically shows the inhibitory effect of compound SLB-605 over two different rhabdomyosarcoma (RMS) cell lines, RD and RH4. Dimethyl sulfoxide (DMSO) is used as a control. N1 and n2 represent two independent experiments. Results are expressed as proliferation values (%), thus the lower value the better result.

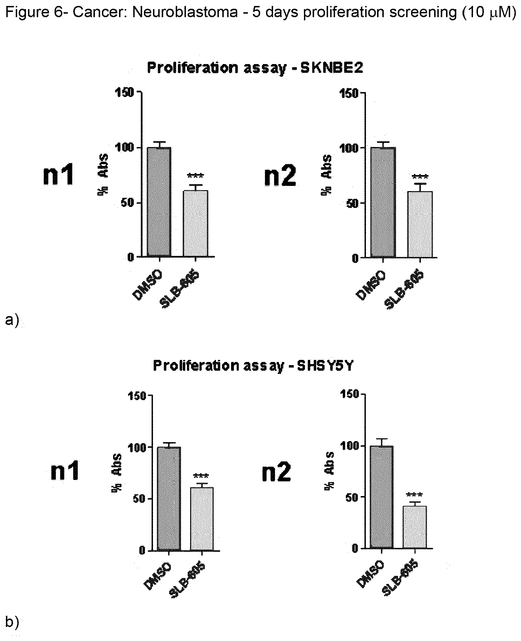

Figure 6 graphically shows the inhibitory effect of compound SLB-605 over two different neuroblastoma (NB) cell lines, SKNBE2 and SHSY5Y. Dimethyl sulfoxide (DMSO) is used as a control. N1 and n2 represent two independent experiments. Results are expressed as proliferation values (%), thus the lower value the better result.

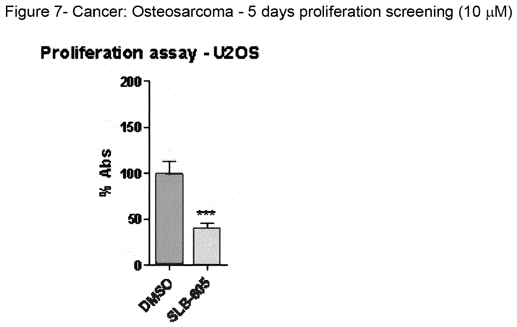

Figure 7 graphically shows the inhibitory effect of compound SLB-605 over a osteosarcoma (OS) cell line, U2OS. Dimethyl sulfoxide (DMSO) is used as a control. Results are expressed as proliferation values (%), thus the lower value the better result.

## Claims

1. A compound having formula I: 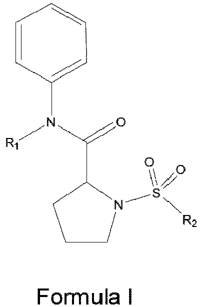 and stereoisomers and pharmaceutically acceptable salts thereof, wherein
R¹ is hydrogen; alkyl optionally substituted with Het; benzoyl optionally substituted with alkyl or cyano; or Het;
R² is aryl optionally substituted with 1, 2, or 3 substituents each independently selected from alkyl, halo, cyano, amide, and carboxyl; or benzyl optionally substituted with 1, 2, or 3 substituents each independently selected from alkyl, halo, cyano, amide, and carboxyl; wherein each Het as a group or part of a group is a monocyclic ring with 5 or 6 atoms; or a bicyclic ring structure consisting of a 6 membered ring fused to a 4, 5, or 6 membered ring; each of the rings being saturated, partially unsaturated, or completely unsaturated, wherein at least one of the rings contains 1 to 4 heteroatoms each independently selected from nitrogen, oxygen and sulphur; and any of the rings is optionally substituted with one, two or three substituents each independently selected from the group consisting of halo, hydroxyl, nitro, cyano, C₁₋₆alkyl, and C₁₋₆alkoxy.
for use as a calcineurin inhibitor in the prevention and/or treatment of a disease or disorder related thereto.

2. The compound for use according to claim 1, wherein
R¹ is hydrogen; C₁₋₆alkyl optionally substituted with Het; benzoyl optionally substituted with C₁₋₆alkyl or cyano; or Het.
R² is aryl optionally substituted with 1, 2, or 3 substituents each independently selected from C₁₋₆alkyl, halo, cyano, amide, and carboxyl; or benzyl optionally substituted with 1, 2, or 3 substituents each independently selected from C₁₋₆alkyl, halo, cyano, and carboxyl.
wherein each Het as a group or part of a group is a monocyclic ring with 5 or 6 atoms; or a bicyclic ring structure consisting of a 6 membered ring fused to a 5 or 6 membered ring; each of the rings being saturated, partially unsaturated, or completely unsaturated, wherein at least one of the rings contains 1 to 4 heteroatoms each independently selected from nitrogen, oxygen and sulphur; and any of the rings is optionally substituted with one, two or three substituents each independently selected from the group consisting of halo, hydroxyl, cyano, and C₁₋₆alkyl.

3. The compound for use according to any one of claims 1-2, wherein
R¹ is hydrogen; methyl optionally substituted with Het; or benzoyl optionally substituted with cyano;
R² is aryl optionally substituted with 1, 2, or 3 substituents each independently selected from methyl, halo, cyano, amide, and carboxyl; or benzyl optionally substituted with 1, 2, or 3 substituents each independently selected from methyl, halo, and cyano.
each Het as a group or part of a group is a bicyclic ring structure consisting of a 6 membered ring fused to a 5 or 6 membered ring; each of the rings being partially unsaturated; wherein at least one of the rings contains 1 to 4 heteroatoms each independently selected from nitrogen, oxygen and sulphur.

4. The compound for use according to any one of claims 1-3, wherein
R¹ is selected from: 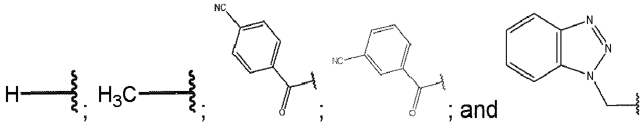
R² is selected from:

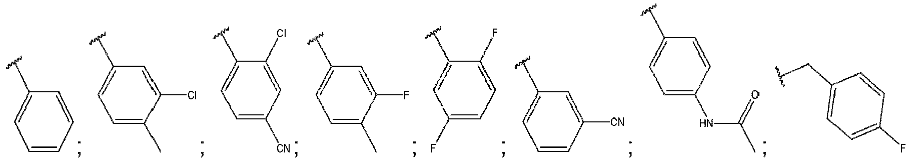

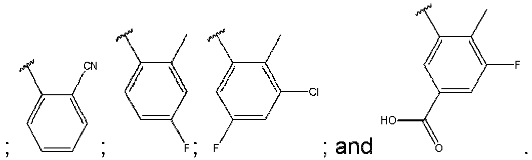

5. The compound for use according to any of claims 1 to 4, wherein said disease or disorder is selected from an autoimmune disease, organ transplant rejection, cancer, heart-related disease or disorder, muscle-related disease or disorder, and neurological disease or disorder.

6. The compound for use according to claim 5, wherein said autoimmune disease is selected from arthritis, anemia, psoriasis, dermatitis, urticaria, sclerosis, multiple sclerosis, inflammatory bowel disease, Crohn's disease, colitis, endocarditis, endometriosis, episcleritis, respiratory distress syndrome, meningitis, iritis, choroiditis, spondylitis, sudden hering loss, rhinitis, encephalitis, uveitis, autoimmune mocarditis, leukocyte adhesion deficiency, lupus, systemic lupus erythemotosus, diabetis mellitus, tuberculosis, sarcoidosis, granulomatosis, vasculitides, autoimmune aplastic anemia, autoimmune neutropenia, CNS inflammatory disorders, Bechet's or Behcet's disease, pemphigoid, pemphigus, Reiter's disease, nephritis, IgM polyneuropathies, thrombocytopenia, hypothyroidism, hypoparathyroidism, thyroiditis, Grave's disease, polyglandular syndromes, paraneoplastic syndromes, encephalomyelitis, myasthenia gravis, autoimmune hepatitis, Guillain-Barre syndrome, Berger's disease, dermatosis, Celiac disease, amylotrophic lateral sclerosis, coronary artery disease, autoimmune hearing loss, polychondritis, paraneoplasic syndrome, channelopathies, epilepsy, migraine, autism, fibromyalgia, multiple endocrine failure, presenile dementia, Chagas' disease, rheumatic fever, recurrent abortion, erythema multiforme, alveolitis, leprosy, malaria, leishmaniasis, dengue, endocarditis, fibrosis, cyclitis, human immunodeficiency virus (HIV) infection, Alzheimer's disease, Epstein-Barr virus infection, keratoconjuctivitis, bronchitis, chronic obstructive airway disease, idiophatic facial paralysis, chronic fatigue syndrome, febris rheumatica, hypogonadism, pancreatitis, vitiligo, acquire immune deficiency syndrome, multiple organ injury syndrome, autoimmune atrophic gastritis, rheumatic diseases, myocarditis, nephrotic syndrome, sinusitis, eosinophilia, hyperalgesia, meningitis, diabetic disorders, valvulitis, Addison's disease, juvenile dermatomyositis, scleroderma, periorificial dermatitis, steroid-resistant nephrotic syndrome and Focal segmental glomerulosclerosis (FSGS).

7. The compound for use according to claim 6, wherein said autoimmune disease is selected from arthritis, anemia, psoriasis, dermatitis, sclerosis, multiple sclerosis, meningitis, rhinitis, lupus, diabetis mellitus, CNS inflammatory disorders, pemphigus, nephritis, autoimmune hepatitis, Celiac disease, migraine, fibromyalgia, endocarditis, coronary artery disease, epilepsy, Chagas'disease, rheumatic fever, malaria, Alzheimer's disease, Epstein-Barr virus infection, bronchitis, chronic obstructive airway disease, chronic fatigue syndrome, pancreatitis, acquire immune deficiency syndrome, multiple organ injury syndrome, myocarditis, meningitis, diabetic disorders Addison's disease, juvenile dermatomyositis, scleroderma, periorificial dermatitis, steroid-resistant nephrotic syndrome and Focal segmental glomerulosclerosis (FSGS).

8. The compound for use according to claim 5, wherein said disease is cancer.

9. The compound for use according to claim 8, wherein said cancer include sarcoma, blastoma, neurological cancer, Hedgehog pathway related cancer, rhabdomyosarcoma, neuroblastoma, medulloblastoma, intracranial cancers, brain cancers, including brain metastases, glioma, glioblastoma, pediatric brain tumors, embryonal tumors, pineoblastoma, oligodendroglioma, pituitary tumors, Ewing's sarcoma, meningioma, ependymoma, craniopharyngioma, choroid plexus carcinoma, astrocytoma, acoustic neuroma, and soft tissues sarcoma.

10. A compound of formula I, wherein:
R₁ is: 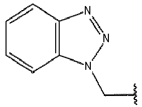
R₂ is: 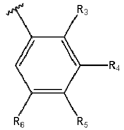 wherein:
R₃, R₄, and R₆ are independently H, alkyl, halo or carboxyl;
R₅ is H, halo, or cyano.

11. The compound according to claim 10, wherein:
R₃, R₄, and R₆ are independently H, C₁₋₃alkyl, halo, or carboxyl;
R₅ is H or halo; and
wherein at least two of R₃, R₄, R₅ and R₆ are different from hydrogen.

12. The compound according to any one of claims 10-11, wherein:
R₃ is methyl;
R₄ is H, F, or CI;
R₅ is H or F;
R₆ is H, F, or carboxyl;
wherein at least two of R₄, R₅ and R₆ are different from hydrogen; and
at least one R₄, R₅ and R₆ is halogen.
